# EUROPEAN PATENT APPLICATION

(11) **EP 1 186 299 A1**
(43) Date of publication of application: **13.03.2002**
(21) Application number: 00203156.5
(22) Date of filing: 12.09.2000
(51) Int. Cl.: A61K 38/17, A61K 38/39, A61P 9/10, A61K 48/00, A61K 39/39, G01N 33/68, G01N 33/92, A23L 1/29

(54) **The diagnosis, prevention, and/or succesful treatment of atherosclerosis, infectious diseases, and disturbances in the immune system**

(71) Applicant: Universitair Medisch Centrum Utrecht, 3584 CX Utrecht (NL)
(72) Inventor: Cabezas, Manuel Castro, 3705 GG Zeist (NL); van Dijk, Hans, 3732 HC De Bilt (NL)
(74) Representative: Prins, Adrianus Willem

(57) **Abstract**

Complement is recognized as an important, humoral defense system involved in the innate (nonspecific) recognition and elimination of microbial invaders, other foreign particles or molecules, and antigen-antibody complexes from the body. The present invention makes use of the surprising notion that the handling of lipids by the body, rather than its antimicrobial activity, is the primary and most ancient function of the complement system. Consequently, atherosclerosis as observed in disorders associated with disturbed lipid metabolism (familial combined hyperlipemia [FCHL], postprandial hyperlipidemia, hypertriglyceridemia with low levels of HDL cholesterol, and insulin resistance associated with type-II diabetes and obesity), must be ascribed to either genetic or acquired defects in ancient (activatory and/or regulatory) complement components. Based on this new insight, novel preventive measures and treatment modalities of disturbed lipid metabolism are introduced.

Other implications of the same invention, based on the notion that lipoproteins and lymphocytes share the lymph pathway to arrive in the blood circulation, are that the lipid metabolising system may be employed to effectively manipulate the immune system. Based on this aspect of the invention, novel oral vaccination and oral immunomodulation strategies are introduced as well.

## Description

The invention relates to the laboratory diagnosis, prevention, and/or treatment of atherosclerosis and underlying or associated diseases.

According to the classical view, atherosclerosis is a condition ultimately leading to the narrowing of blood vessels, consequently impaired circulation, and therefore restricted oxygenation of tissues [1]. If this process occurs in the heart vessels (coronary arteries), consequences are the clinical conditions of angina pectoris and myocardial infarction; in the brain, atherosclerosis leads to cerebrovascular accidents; in the legs, the clinical presentation is claudicatio intermittens. Classical risk factors resulting in atherosclerosis are: obesity, hypertension, smoking, diabetes, male gender, fasting hyperlipidemia, and especially increased cholesterol concentrations [2]. Novel risk factors have emerged during the last decennia like: hyperhomocysteinemia, hypertriglyceridemia with low HDL cholesterol concentrations, postprandial hyperlipidemia, the insulin resistance syndrome, and a positive family history for cardiovascular disease [3].

According to epidemiological surveys, coronary heart disease (CHD) is the leading cause of death in western societies. In the United Kingdom in 1987, 31% of all deaths in males (280.177 total deaths in men) and 24% in females (total number: 286.817) were due to CHD. More than one quarter of CHD deaths in men (total CHD mortality in men: 86.978) occurred before the age of 65 years. In women (68.257 CHD deaths), the vast majority (alsmost 75%) occurred at ages beyond 75 years. The Dutch situation is similar and representative for other countries in Western society. In The Netherlands in 1997, there were 135.783 deaths in total (67.242 males and 68.541 females). In men, 37% of total mortality (24.664 deaths) was due to CHD and in women 38% (25.881 deaths). From 1972 to 1997, mortality due to CHD in The Netherlands decreased by 44% (age-corrected); however, hospital admissions to CHD increased by 53% (data in file of the 'Centraal Bureau voor de Statistiek'). Similar data have been reported for *e*.*g*. different populations in the USA where mortality due to coronary sclerosis declined, but the incidence of myocardial infarction was unchanged or even increased [4]. This decrease in CHD-associated mortality is probably to be ascribed to improved care in coronary-care and intensive-care units [4]. In addition, the early recognition of the above-mentioned risk factors for CHD and improved treatment of these risk factors may have led to increased survival in patients at risk.

The classical drugs for the treatment of these risk factors are cholesterol-lowering drugs (mainly statins) [5], and drugs aiming at the reduction of blood pressure like angiotensin-converting-enzyme inhibitors [6]. The effects of life-style change to reduce body weight and stopping smoking have been disappointing so far, although their impact has not well been established on a population basis. Improvement of regulation of diabetes has resulted in decreased morbidity (less amputations, less diabetics with end-stage renal failure necessitating dialysis, and less diabetics becoming blind) [7, 8], but the incidence of cardiovascular disease in diabetics did not decrease by these measures [8, 9].

Many investigators point at the need for the recognition of concealed risk factors for CHD in diabetes (and obesity) and a more aggressive treatment of these factors which will probably result in improved outcome [3]. Moreover, land-mark trials with lipid-lowering drugs in secondary and primary prevention settings have resulted in significantly decreased mortality in the treated patients (30% risk reduction) [3], but there were still significant numbers of patients which could not be saved from fatal outcome by these drugs. Therefore, the identification of additional risk factors and the development of novel therapeutic interventions is expected to result in a significant reduction of total mortality due to CHD.

One of the recently recognized mechanisms in the development of atherosclerosis is inflammation [10]. Several studies have demonstrated that slightly elevated concentrations of C-reactive protein (CRP; a well-known acute-phase reactant) predict coronary events in middle-aged men and women [11-13], but also in the elderly [14], thus providing support for this novel concept of atherosclerosis.

We have developed a novel, mechanistic concept providing an explanation for the insufficient protective effects of lipid-lowering drugs, the persistently high incidence of coronary heart disease in western societies, and the relationship with markers of inflammation like CRP. In addition, a novel approach for the diagnosis, prevention, and/or therapy of atherosclerosis and underlying or related disease(s) is presented which may be implemented at a large scale in combination with current strategies to lower mortality and morbidity by CHD.

The present invention has as an object to provide new and improved manners of prevention and/or treatment of atherosclerosis and underlying / related diseases. The invention further seeks to provide new and improved manners of determining the occurrence (diagnosis) of atherosclerosis and related diseases, in particular those which are associated with disturbed lipid metabolism.

It has been postulated that atherosclerosis is associated with an impaired clearance of chylomicron remnants. In accordance with the invention, it has surprisingly been found that said clearance of chylomicron remnants is positively regulated by the complement system that is to say by the most ancient complement activation pathways, the 'lectin' and 'alternative' pathways. Moreover, the invention includes that the absence of low serum levels of the intercellular matrix proteins vitronectin and/or clusterin, which function as regulators of the 'terminal' or 'lytic' pathways of complement, may lead to decreased intravascular integrity of chylomicron remnants.

Accordingly, the invention relates to the use of purified or enriched physiologic complement components, physiologic complement regulators and/or extrinsic complement modulators of natural (*e*.*g*. plant-derived), synthetic, or semisynthetic origin in the prevention and/or treatment of atherosclerosis, but also in the treatment of infectious diseases, and disturbances in the immune system.

Because a thorough and mechanistic insight has now been achieved, it has been found possible to develop novel diagnostic tools and to formulate specific and highly effective primary and secondary prevention strategies for disturbances leading to atherosclerosis. Dependent on what is (are) the weakest link(s) in the specific pathways of the complement system in an individual patient, a physician can, based on the considerations of the invention, intervene in the complement system of the patient in order to prevent and/or treat manifestations of disease.

### Introduction into the complement system

The complement system [15] is an enzymic signalling system present in blood that is involved in the early recognition and clearance of foreign bodies and antigen-antibody complexes (immune complexes) from the circulation and tissues. Clearance is largely mediated by the mononuclear phagocytic system (monocytes and macrophages) present in the liver, spleen, lymph nodes, or the affected tissues themselves. In contrast to mononuclear phagocytes, polymorphonuclear phagocytes (PMNs) are relatively inefficient in eliminating foreign material, at least in the absence of antibodies.

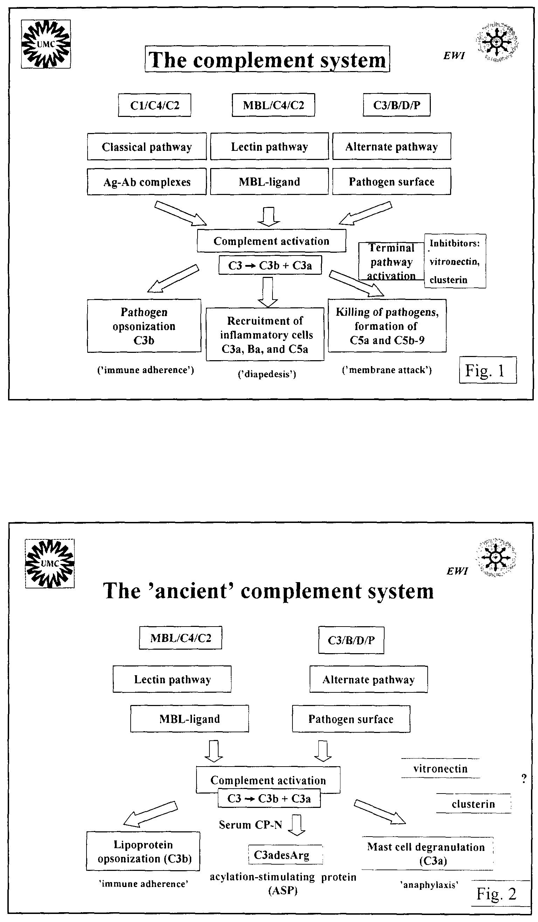

Upon the recognition of foreign material in tissue or blood, the most crucial and abundant complement component, C3, becomes activated, i.e. is converted into C3b and C3a (Fig. 1) C3a is in fact the N-terminus (of 77 amino acids) of the α chain of C3, whereas C3b - consisting of the C-termini of the α and β chains - binds covalently to sugar OH groups or protein NH₂ groups on the substrate via ester and amide bonds, respectively. C3b and its inactivated form (C3bi) are opsonins, which implicates that they promote the phagocytosis and inactivation of foreign bodies within phagocytes (in the absence of antibodies predominantly by monocytes and macrophages) via their complement receptors CR1, CR3, and possibly also CR4. C3 split product C3a is a spasminogen and anaphylatoxin, which induces the release of histamine from basophilic cells, including tissue mast cells and basophilic granulocytes. Histamine, in turn, enables phagocytes to leave the blood vessels in order to arrive at the site of complement activation, i.e. the accumulation site of foreign material or immune complex. In blood, C3a is rapidly (in about 15 min.) inactivated by serum carboxypeptidases which remove C-terminal arginine, resulting in the generation of C3adesArg. The most prominent serum carboxypeptidase (SCP) in blood is the constitutively expressed sCP-N [16, 17]. All other sCP types are inducible and are less abundant than the N type.

In primates, the clearance of immune complexes by the liver and spleen follows a special pathway, i.e. transportation via erythrocytes, which carry a restricted number of CR1 molecules on their surface [18]. This phenomenon has been referred to as 'immune adherence'. Erythrocytes of non-primate species are CR1 negative and consequently do not mediate the transport of immune complexes to these organs.

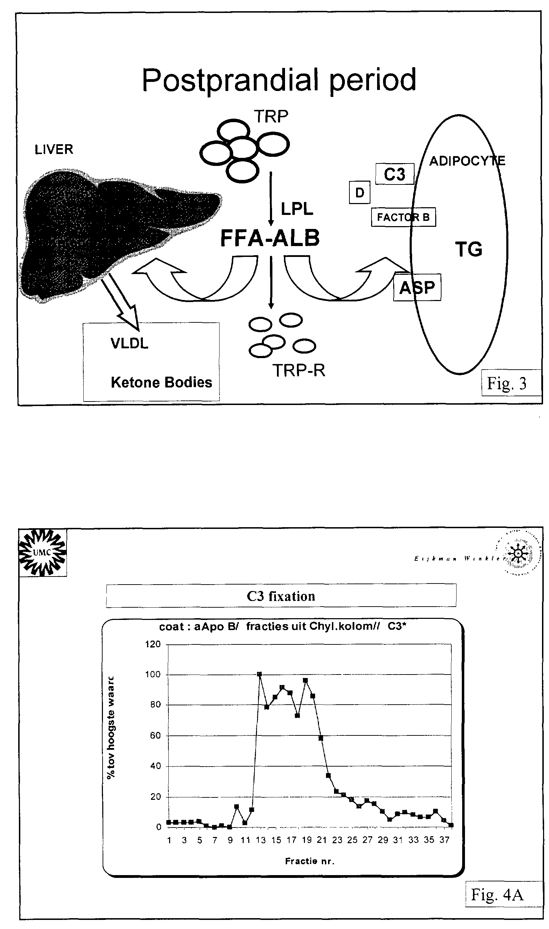

The conversion of C3 into C3b and C3a is mediated by so-called C3 convertases, which are the products of three different complement activation pathways (Fig. 1). These pathways include, in order of descending evolutional age, the so-called 'lectin' pathway (LP), the 'alternative' pathway (AP) which is also known as the 'amplification loop', and the relatively young 'classical' pathway (CP). Furthermore, there is also a so-called 'terminal' or 'lytic' pathway attached to the complement system, which can destabilise membranes by pore formation, resulting in the killing of e.g. Gram-negative bacteria, virus-infected body cells, or even tumour cells. Phylogenetic studies have pointed out that the lectin and alternative pathways are by far the most ancient complement activation pathways (about 700 million years; Fig. 2), whereas the classical and lytic pathways are relatively young (400 - 350 million years) [19].

### The lectin pathway of complement activation

Activation of the lectin pathway (LP) starts with the recognition and binding of foreign bodies by a serum lectin [20], called mannose-binding lectin (MBL). MBL is a high-molecular-weight, sugar-binding protein, present in minute amounts (about 2 µg per ml) in blood plasma [21]. MBL belongs - together with C1q, lung surfactant proteins A (LspA), and LspD - to the family of the collagenous lectins (collectins) [22]. Upon binding, two MBL-associated proteins s(MASP-1 and MASP-2) become coordinately activated, ultimately leading to the generation of the active form of MASP-2, the LP-dependent C4/C2 convertase. This convertase, which has C4 and C2 as its natural substrates, generates essentially three products: C4bC2a and split products C4a and C2b. Like C3b, the C4b portion of C4bC2a binds covalently to its substrate (*e*.*g*. polysaccharides or (glyco) proteins on bacteria) via ester or amide bonds, whereas two split products, C4a and C2b, are released in the fluid phase. Substrate-bound C4bC2a is a C3 convertase, causing the conversion of C3 into C3b and C3a. Like C3a, C4a is a spasminogen and anaphylatoxin (histamine liberator), whereas C2b has kinin-like activity.

MBL mediates the recognition of foreign bodies by its 6 identical sugar-binding moieties with specificity for mannose, N-acetyl-glucosamine, and fucose [20]. This makes sense, because microbial pathogens like fungi, yeasts, and *Mycobacteria* carry relatively high amounts of mannose, while peptidoglycan of Gram-positive bacteria contains N-acetyl-glucosamine as one of its major brigstones. An interesting additional fact in this context is that IgA is also heavily mannosylated, which implies that this antibody uses the lectin pathway to reach opsonization and elimination of foreign particles or molecules from the body.

### The alternative pathway of complement activation

Until the discovery of the *lectin pathway* in 1989 [21], the alternative complement pathway (AP), first described in 1956, was considered the most ancient complement activation route [19]. The main function of this alternative complement pathway is to amplify the number of C3-converting sites on the substrate of complement activation: the foreign body or the immune complex. This means that, once 'non-self material has been identified by MBL and activation of the lectin pathway has consequently taken place, the LP-dependent C3 convertase present on the substrate will be amplified by AP-dependent C3 convertases in the following manner(Fig. 1): Substrate-bound C3b, generated by the LP-dependent C3 convertase (C4bC2a), will bind AP component factor B which, in turn, will be activated to Bb by AP component factor D (also known as adipsin) [15] to form the AP-dependent C3 convertase (C3bBb). Along with the formation of this new C3 convertase, the factor-B part loses a split product called Ba. The enzymic function of the AP-dependent C3 convertase is considerably stabilised upon the binding of AP component 'properdin' (factor P). Split product Ba is a leukotaxin, which helps to direct the movement of phagocytes to the site of complement activation (primary inflammation site).

The net result of AP activation is an increase in the number of C3b and inactivated C3b (C3bi) moieties on the substrate, which promote the recognition and clearance of foreign bodies and immune complexes by, predominantly, mononuclear phagocytes (monocytes/ macrophages).

### The classical pathway of complement activation

The classical complement pathway (CP) is generally considered the youngest complement activation route, since it is dependent on antibodies (IgM and IgG), which appeared relatively late in phylogeny (from about 350 million years ago; 19). The CP is very similar to, and therefore probably derived from the ancient *'lectin'pathway,* since the first CP component (C1; consisting of a complex of the collectin C1q and two MASP-like proteins called C1r and C1s) is both phenotypically and functionally very much related to the MBL/ MASP-1/ MASP-2 complex. In addition, the classical pathway involves lectin-pathway complement components C4 and C2. Like the sugar-bound MBL/MASP-1, 2 complex, C1 (composed of C1q, C1r, and C1s) bound to IgM-or IgG-type immune complexes becomes coordinately activated to form a C1-esterase which has C4 and C2 as its natural substrates and which gives rise to the generation of CP-dependent C3 convertases, which are identical to LP-dependent C3 convertases (substrate-bound C4bC2a complexes).

### The terminal or 'lytic' complement pathway

When a newly formed C3b molecule does not bind to the substrate directly, but to another, substrate-bound C3 convertase (C4bC2a or C3bBbP), triple or quadruple complexes consisting of C4bC2aC3b or C3bBbC3bP are formed. These complexes have C5-converting activity indicating that they are able to split complement component C5 into C5b and C5a. This is the starting point of the so-called 'terminal 'or 'lytic' complement pathway. Like Ba, C5a is a leukotaxin, but more potent than Ba. C5b forms a complex with C6 and C7, the resultant of which is a soluble C5b-7 complex, which has affinity for membranous bilayers. Upon insertion into a membrane of *e*.*g*. a Gram-negative bacterium, complement component C8 will bind to the complex, which results in a new enzyme, the membrane-bound C9 polymerase (C5b-8). Under the influence of one C5b-8 complex, some 13 C9 molecules become polymerised, resulting in a cylindric pore in the membrane that is under attack. Depending on the total number of membrane-bound poly-C9 pores and on whether the bacterium is encapsulated or not, the Gram-negative bacterium will either be killed or be able to resist and survive membrane attack.

### Complement regulation and complement regulators

In order to prevent unwanted activation of the complement cascade *e.g.* by cells of the body itself, complement activation on homologous cells is heavily regulated by both cell-bound complement inhibitors and regulators in the fluid phase.

The most important **soluble** regulators are:

### For the lectin pathway:

C1 inhibitor (C1INH), α₂-Macroglobulin (α2M), and C4-binding protein (C4BP), which interfere with the formation of the LP-dependent C4/C2-convertase (activated MBL/MASP1/MASP2 complex) and the subsequent activation of C4 and C2 [22].

### With regard to the alternative complement pathway:

Factor H and factor H-like molecules, acting at the level of factor B binding to target-bound C3b (preventing the formation of AP-dependent C3 convertases),

C3b inactivator (factor I), acting in conjunction with factor H, to convert C3b in its enzymatically inactive, but as opsonin still active form C3bi.

### As to the classical complement pathway:

C1INH, acting at the level of activated C1 (the C1 esterase), and

### With regard to the terminal complement pathway:

Vitronectin (S protein) and clusterin (the latter also being known as apolipoprotein J, because it has been described in association with HDL). These proteins act at the level of C5b-7 complexes, preventing their insertion into bilayer membranes and inhibiting C9 polymerization and consequently, the lysis of bacteria, viruses, body cells, and presumably also chylomicrons and their remnants.

### Cell-bound complement regulators include:

Complement receptor 1 (CR1) which has a factor-H-like co-enzyme function versus factor I; CR1 is present on phagocytes, platelets, but also as a carrier protein on erythrocytes [15],
Decay-accelerating factor (DAF = CD55) and membrane cofactor protein (MCP = CD46), both acting at the level of AP activation,
Homologous restriction factor with 20-k molecular mass (HRF20 = CD59) and HRF60, both inhibitory at the level of C9 polymerase (C5b-8) formation, and
Sialic acid, which acts similar to CD55 and CD46 at the level of the AP-dependent C3-convertase formation, but also on C9 polymerization.

### Activation or down-modulation of complement and their effects on the immune response

Apart from the physiological activatory and regulatory complement components as mentioned above, different substances of bacterial, plant, animal, or (semi)synthetic origin have been identified as either activators or down-modulators of the complement cascade(s). These components include bacterial lipopolysaccharides [23-29], β-glycyrrhetinic acid [30], phytosterols [31], bovine conglutinin [1], and polymeric substances like dextran sulphate and glucans [32-34]. After having had a long history as alternative-pathway activators, bacterial lipopolysaccharides have recently been recognised as potent activators of the lectin pathway, in addition to which the alternative pathway may serve as an 'amplification loop' [22].

Likewise, β-glycyrrhetinic acid, as a presumable activator of C4 [30], is likely to activate the lectin pathway, while the phytosterols with as most important repesentatives β-sitosterol, stigmasterol, and campesterol, have been shown to activate the alternative complement pathway [31]. Dextran sulphate, on the other hand, functions as an acceptor site for alternative-pathway regulatory protein factor H (β1H), in this way facilitating the alternative-pathway-mediated activation of C3 and subsequent deposition of C3b.

Based on their complement-activating capacity, a number of these substances, including bacterial lipopolysaccharides, dextran sulphates, and glucans, as well as lipidated muramyl-dipeptides [35, 36] and lipophilic quaternary ammonium compounds like dimethyldioctadecyl ammonium bromide [37] show potent immunological adjuvant activity, which means that they are able to stimulate antigen-specific T- and B-cell responses. Indirectly, these results implicate that lipid-soluble immunological adjuvants, if administered orally together with oil, will not only influence the immune response, but presumably also change the fate of lipoproteins in the body. This must be ascribed to the fact that the immune system and lipid metabolism share the lymph pathway. It is even conceivable that oral administration of a lipid-soluble vaccine administered in combination with oil and a lipid-soluble immunological adjuvant will not only stimulate 'classical' i.e. central T- and B-cell responses, but also - and more likely - to induce vaccine-specific, mucosal IgA production.

### The physiology of lipid metabolism

Under physiologic conditions, about 90% of the ingested fat (triglycerides) is taken up by the epithelial cells of the small intestine, resulting in the generation of intestinally-derived triglyceride-rich lipoproteins, called chylomicrons [38]. These chylomicrons are transcytosed through the epithelial cells and delivered at their basolateral side to the subepithelial interstitium. The structure of chylomicrons is safe-guarded by a large, highly glycosylated protein, called apolipoprotein B48 (apo B48), of which the most dominant glycosyl residues are: mannose (17.8%), N-acetyl-glucosamine (16.8%), galactose (13.4%), and fucose (3.4%) [39]. Apo B48 is the 5' splice product of a larger *apob* gene which - in human intestinal epithelial cells - is posttranscriptionally modified by a unique editing enzyme. This modification results in a premature stopcodon leading to the translation of only 48% of the *apob* mRNA. Since the human liver lacks the editing enzyme, *apob* transcription in the liver results in the synthesis of full-length apo B100 [40].

From the subepithelial interstitium, chylomicrons are collected in tissue fluid (lymph). Via lymph vessels, they are transported to subsequent draining lymph nodes and, through the thoracic duct and the left subclavian vein, they finally arrive in the blood stream. Once in the circulation, chylomicrons are rapidly converted into chylomicron remnants by the action of vascular- endothelium-associated lipoprotein lipase (LPL) [41].

Chylomicrons and chylomicron remnants are subsequently cleared efficiently by the liver from where they can undergo bile-mediated excretion via the stools. However, the efficiency of the process of chylomicron and chylomicron-remnant targeting to the liver is far from understood, while the subsequent hepatic clearance of these triglyceride-rich particles has not completely been elucidated either. In the liver, it involves at least the activity of the hepatic triglyceride lipase (HTLG) [38], interaction with specific apo E receptors [41], and non-receptor binding to the cellular surface in the hepatic space of Disse. Several local receptors may be involved including low-density-lipoprotein receptor-related protein/α₂ -Macroglobulin receptor (LRP-α₂M) [42,43], a parenchymal liver cell 'chylomicron remnant receptor' [44], the asialoglycoprotein receptor [45], the lipolysis-stimulated receptor [46], and the LDL receptor [47]. Recently, the VLDL receptor, a new member of the LDL receptor supergene family, which is not present in the liver, has been recognised as a physiological receptor for chylomicron remnants [48].

Cholesterol, delivered to the liver by chylomicrons and chylomicron remnants, is largely re-secreted into the circulation after incorporation into very-low density proteins (VLDL). This cholesterol is further employed by the adrenals and genitals as a skeleton for their steroid-hormone synthesis.

Free fatty acids (FFA) arising from the interaction of chylomicrons with endothelial LPL are transported over the mucosa towards subendothelial fat cells (adipocytes) in which they become re-esterified into intracellular triglycerides (Fig. 3). The uptake and incorporation of FFA into adipocytes is under the positive control of a hormone called acylation-stimulating protein (ASP) [49,50]. Partially hydrolyzed chylomicrons, known as chylomicron remnants, are present in blood in different sizes.

Similarly to the hydrolysis of triglycerides in chylomicrons, VLDL may become VLDL remnants (= intermediate-density lipoproteins; IDL) under the influence of LPL, in this case under the positive and negative control of two other apolipoproteins, known as apoCII and apoCIII [11], respectively. Moreover, IDL are enriched in apo E which functions as the ligand for the hepatic LDL receptor and the hypothetical 'remnant receptor'. Under physiological conditions, IDL are taken up by these receptors in the liver, by which organ the lipoproteins are degraded and cholesterol is removed from the body by excretion into the bile.
**In conclusion:** the metabolic pathways of the intestinally- and liver-derived triglyceride-rich particles in blood, chylomicrons and VLDL, respectively, and their remnants have been partially identified. These pathways have been shown to have certain overlaps. In particular, the - under physiological conditions - very efficient targeting of chylomicrons and chylomicron remnants to the liver and their clearance, need further elucidation.

The present invention is *inter alia* based on the consideration that the clearance of chylomicrons and their remnants is under the positive regulation of the most ancient activation pathways of the complement system, the 'lectin' and 'alternative' pathways, while vitronectin and clusterin have as important function the maintainance of the integrity of chylomicrons and chylomicron remnants, by down-regulating the 'lytic' complement pathway.

### Aberrant lipid and/or free-fatty-acid metabolism

Chylomicron remnants are potentially atherogenic particles due to their ability to directly induce foam-cell formation, without any modification [38, 51]. The latter is in contrast to the situation with low-density lipoprotein particles (LDL), which must be oxidised before they induce transformation of mononuclear phagocytes (monocytes/macrophages) into foam cells [52]. The background of this different processing is that mononuclear phagocytes have an LDL receptor by which they are able to internalise and subsequently degrade native LDL. This uptake and intracellular degradation of LDL is, in turn, controlled by the intracellular free cholesterol concentration, which down-regulates LDL receptors, as soon as the cholesterol level reaches a given threshold. In contrast, oxidised LDL are taken up via 'scavenger' receptors, which are not subject to down-regulation.

Since chylomicrons, VLDL, and their remnants compete for the same metabolic pathways, in patients with delayed clearance there may be a temporary accumulation of chylomicrons and chylomicron remnants in the circulation, which obviously contributes to the process of atherogenesis [38, 51]. Such situations are likely to occur in patients with familial combined hyperlipidemia (FCHL), type-2 diabetes mellitus, insulin resistance, and obesity [51-56]. Enhanced plasma VLDL levels in these situations have been associated with delayed clearance of chylomicron remnants as studied after a single oral fat load [51-59].

Similar mechanisms are involved in situations in which the clearance of remnant particles is impaired due to mutations in the apo E ligand gene (type III hyperlipidemia = familial dysbetalipoproteinemia) [53-54, 60], the LDL receptor (familial hypercholesterolemia; FH) [58], familial defective apo B100 (FDB) [61] and after menopause [62]. In these conditions, all associated with the development of (premature) atherosclerosis, a delayed clearance of chylomicron remnants has been established due to an impaired binding to receptors in the liver. Another aspect of these diseases is that the hepatic VLDL synthesis is upregulated resulting in increased blood levels of VLDL as well [38, 53-58].

Other disorders associated with impaired remnant clearance are apo CII deficiency, (partial) lipoprotein lipase (LPL) deficiency, and hepatic triglyceride lipase (HTGL) deficiency [63-65]. In these disorders, the conversion of triglyceride-rich particles into their remnants is delayed, leading to an accumulation in the circulation of triglyceride-rich particles of different sizes and triglyceride content.

In many endocrinological disorders like hypothyroidism, growth hormone deficiency, hypercortisolism by endogenous or exogenous corticosteroids, and the postmenopausal state, a decreased clearance of chylomicron remnants has been established when compared with the control situation [54, 62]. Finally, different national and international reports and recent work in Dr Castro Cabezas' laboratory have clearly shown that in patients with premature atherosclerosis and normal fasting plasma lipids (40% of all patients with myocardial infarction below 60 years of age in males and beyond 65 years of age in females), chylomicron-remnant clearance is decreased [66]. It has been hypothetised and it is widely accepted that this may be one of the important mechanisms underlying atherosclerosis in these groups of patients [67-74]. Identification of the underlying defect(s) in these patients and modulation and improvement of their chylomicron-remnant clearance will most likely lead to a reduction of the risk for coronary artery disease and therefore to decreased morbidity and mortality.

### General considerations with regard to the putative relationship between complement and lipid metabolism

Parts of the complement system are phylogenetically older than the immune system [19]; this especially holds true for the *lectin pathway* and the *alternative pathway/amplification loop.* The complement system must therefore have (had) one or more non-immune functions.

Some complement components have other functions and sometimes also different functional names:
1. C1 inhibitor (C1INH) is an inhibitor of complement component C1, but also of other serine esterases as *e*.*g*. in the kallikrein-bradykinin system (kallikrein), clotting factors XIa and XIIa, and fibrinolysis product plasmin [15].
2. Factor D of the alternative pathway is also known as adipsin [15], suggesting a link with lipid metabolism.
3. The inactivated form of anaphylatoxin C3a (desArginated C3a or C3adesArg) is identical to acylation-stimulating protein (ASP) [49, 75, 76], which is known as a positive regulator of free-fatty-acid incorporation into adipocytes.
4. Terminal-complement-pathway inhibitor clusterin is otherwise known as apolipoprotein J (apo J) [77].

It will be understood that the use of the complement components under these names is also encompassed by the invention.

Free fatty acids stimulate adipocytes to synthesise complement component C3 and alternative-pathway components factors B and D [49].

In healthy individuals, there is a linear relationship between total body fat (body mass index) and C3 levels [79-81; and own experiments].

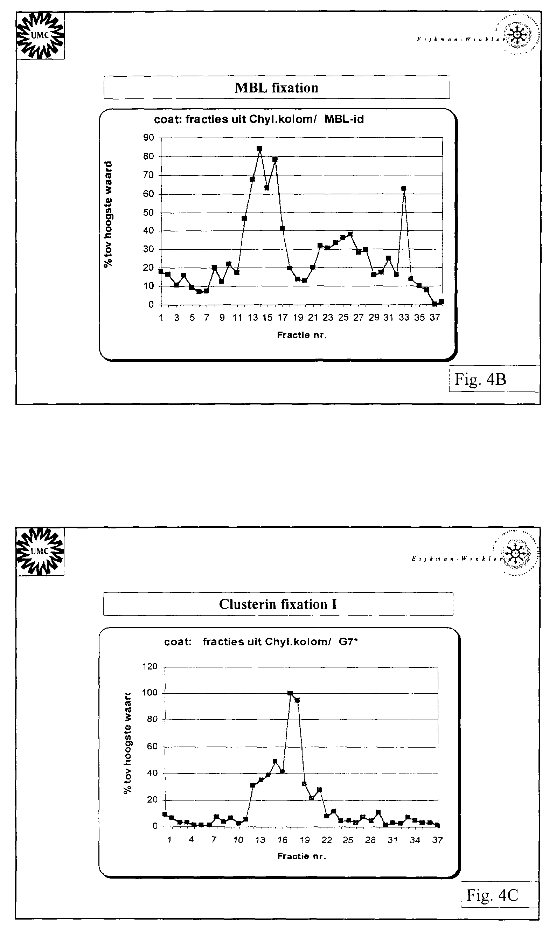

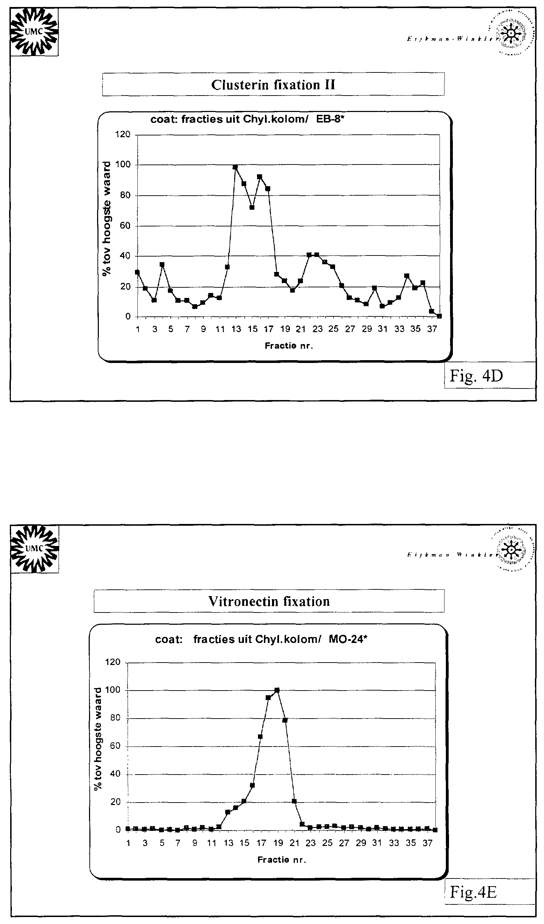

Chylomicrons, isolated from healthy individuals after an oral fat load, carry complement components C3 (i.e. C3b and/or C3bi), mannose-binding lectin (MBL), and the terminal-complement-pathway inhibitors clusterin and vitronectin (own experiments; Figs 4A-E). It is therefore is very likely that chylomicrons activate the lectin pathway and most probably also the alternative pathway (C3, factor B, and factor D synthesis by adipocytes) and, consequently, the terminal complement pathway of the human complement system.

Prominent glycosylation sites of apolipoproteins B48 [39] and B100 [40, 82], present on plasma chylomicrons and VLDL, respectively, match fully with the binding specificities of MBL (mannose, N-acetylglucosamine, and fucose). This strongly suggests that triglyceride-rich particles in blood activate the complement system via their apolipoprotein B, which functions as an intrinsic (i.e. epithelium-derived) activator of MBL, the first component of the lectin activation pathway of the human complement system. Such an intrinsic complement activator may potentially be very harmful, as also evidenced by autoantibodies against C3 convertases (F-42 and C3 nephritic factor) [83] occurring in patients with collagen diseases. This may in particular hold true for subjects with decreased serum levels of terminal-pathway inhibitors vitronectin and/or clusterin [84], since such a situation will allow terminal-complement-pathway activation to occur.

The major histocompatibility complex (MHC) is one of the most ancient systems involved in tissue (in)compatibility as already present in *e*.*g*. sponges (textbook knowledge) [19].

Lectin-pathway complement components C2, C4 variants C4A and/or C4B, and factor B of the alternative pathway are products of the class III region of the MHC [85].

Terminal-complement-pathway activation on triglyceride-rich particles may result in the release of atherogenic lipid material, particularly in patients with a genetic ar acquired deficiency in terminal-complement-pathway regulators vitronectin or clusterin [84].

The binding of terminal-complement-pathway inhibitors vitronectin and clusterin to chylomicrons can teleologically be explained in terms of protection from atherosclerosis.

In primates, the clearance of antigen-antibody complexes, as occurring in systemic autoimmune diseases and as accompanying phenomenon in *e.g.* neoplastic diseases, involves antibody-mediated activation of the complement system via the classical pathway (i.e. via complement components C1, C4, and C2, and ultimately C3). In this way C3b(i)-coated immune complexes are eliminated by the mononuclear phagocytic system in the liver and spleen, after erythrocyte-mediated transportation via the blood stream.; this process is known to be dependent on erythrocyte-bound complement receptors 1 (CR1) [18]. Since primates belong to the only animal species bearing CR1's on their erythrocytes, the erythrocyte-mediated elimination of immune complexes is, so far, restricted to primates [78].

Microbial pathogens in the circulation are also cleared by the mononuclear phagocytic system, but only after MBL (lectin pathway)-mediated or antibody/C1 (classical pathway)-mediated activation of complement components C4, C2, and C3 [75]. This process is known to involve erythrocyte-mediated clearance as well [85]. In older literature, this process was referred to as 'immune adherence').

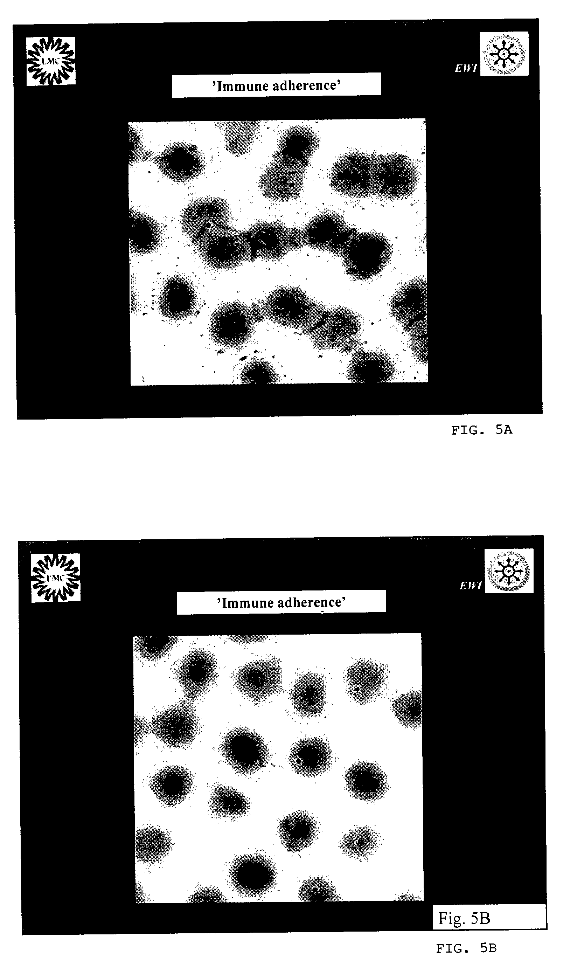

Virtually all erythrocytes of healthy volunteers, 4h after an oral fat load, carry chylomicrons and chylomicron remnants (own experiments; Figs 5A), whereas erythrocytes in the 'vasting' state do not (Fig. 5B). This finding is compatible with the idea of an erythrocyte-mediated elimination of triglyceride-rich particles and can be interpreted in terms of immune adherence of remnant particles and targeting of lipids to the liver and spleen.

Combination of chylomicron (remnant)-induced activation of the lectin pathway, the matching of glycosylation sites of apolipoproteins B48 and B100, and the erythrocyte-mediated elimination of triglyceride-rich particles suggest that increased levels of triglyceride-rich particles in blood, as occurring in FCHL and other disorders associated with atherogenic disturbances of lipid metabolism, may be due to delayed erythrocyte-dependent clearance of chylomicrons and/or VLDL based on:
1. congenital defects in glycosylation of apo B48 and/or apo B100 [86].
2. absolute (homozygous), but also relative or acquired deficiencies of individual complement components of the lectin and alternative activation pathways, as there are:
   - MBL (9% of the population) [87]
   - C4A (defective gene frequency 10-13% the population) [88-92]
   - C4B (defective gene frequency about 17% of the population) [88-92]
   - C2 (rare),
   - C3 (rare),
   - factor B (rare), or/and
   - factor D (rare).
3. deficiences of serum carboxypeptidases which exclude the conversion of C3a into C3adesArg (incidence unknown).
4. absolute (rare) or relative (quite common) deficiencies of complement receptor 1 on erythrocytes as occurring in some patients with systemic lupus erythematosus [93].
5. deficiencies of terminal-pathway regulator vitronectin (4% of the population), which may lead to the lysis of triglyceride-rich particles resulting in unwanted deposition of lipids [84].
6. decreased serum levels of clusterin in association with exacerbations of SLE [77]or with circulating immune complexes accompanying neoplastic diseases (deficiencies of clusterin are rare; <1% of the population) [84].

The incidence of serious cardiovascular disease (37% in 1997 according to the 'Centraal Bureau voor de Statistiek') in the Netherlands expressed as percentage of total numbers of fatal cases per year, matches well with the combined figures for MBL, C4A, C4B, vitronectin, and custerin deficiencies, corrected for the incidence of double and triple deficiencies.

Plant oils regularly contain amphiphilic, complement-activating components that may bypass/correct deficiencies in MBL, C4A, and C4B by activating the alternative complement pathway [31].

The similarity of their elimination pathways suggests that triglyceride-rich particles have to compete with soluble immune complexes and/or microbes for elimination sites on erythrocytes and in liver and spleen, which could explain the disturbed lipid metabolism in *e*.*g*. septic shock.

Several other disorders, the pathogenesis of which has not been clarified yet, may serve as models for the study of the above-mentioned invention. For example, paroxysmal nocturnal hemoglobinuria (PNH), a rare syndrome characterised by episodes of hemolysis and hemoglobinuria, the latter accentuated during sleep, may be one of these models. The primary cause of PNH-associated hemolysis is unknown, but the syndrome has been associated with low levels of complement-regulatory proteins (*e*.*g*. CR1) on blood cells. We postulate that PNH-associated hemolysis is induced by triglyceride-rich particles bound to erythrocytes, giving rise to unregulated complement activation and consequent hemolysis.

A different example may be SLE (systemic lupus erythematosus) in which premature atheroslerosis is a well-known feature which has not been explained so far. We propose that a genetic defect in erythrocyte-bound CR1's [93]or the the binding of immune complexes to these receptors impedes the binding of chylomicron remnants to these same receptors, resulting in impaired erythrocyte-mediated targeting of these atherogenic chylomicron remnants to the liver. This may explain the recently described inefficient chylomicron remnant metabolism in SLE subjects [94].

The physiological routing of ingested lipids via the gut, lymph vessels, and the lymph nodes to the blood stream, implicates that lipidated vaccines intended for oral application, suspended in *e*.*g*. plant oil, may represent a novel and theoretically almost ideal strategy of oral immunisation.

Finally: different plants are known to synthesise mannose- and/or N-acetylglucosamine-specific lectins which may be involved in the transport or accumulation of storage compounds, *e*.*g*. plant oils in the plant [95]. Furthermore, it has been shown in animal studies that mannose- and/or N-acetylglucosamine-binding lectins have potent antilipolytic and lipogenic activities [96].

### Evolutionary concept of the complement system and lipid metabolism

Based on the aforesaid, we postulate that the ancient complement pathways (the lectin and alternative routes) [Fig. 5] originally developed in association with lipid metabolism. In contrast, the evolution of younger complement activation pathways (the classical and terminal pathways, the latter including the regulatory proteins vitronectin and clusterin) was primarily dictated by infectious agents and evolved in close association with the immune system, rather than with lipid metabolism. This is probably the reason why the ancient, lipid-metabolism-related pathways still comprise relatively high percentages of more or less defective components, including mannose-binding lectin (9%; own observations for the Dutch population) [86], C4A (10-13.3%) [88-92], and C4B (7-18.3%) [88-92]. Recently, we finished a study on the incidence of deficiencies of terminal-pathway regulators vitronectin and clusterin (apolipoprotein J) in a population of 416 Dutch volunteers between 3 and 85 of age. The results indicate that the incidence of undetectable vitronectin levels in the study population was about 1%, whereas another 3% was significantly below the lower 0.5th percentile [84]. However, low clusterin levels were rare (below 1% in the total donor population studied), which matches well with the idea that hereditary clusterin deficiency is lethal [84]. Therefore, low clusterin levels must be considered the result of immunologic dysregulation, rather than being genetically determined [77].

Apart from concept that the transportation of lipoproteins in the body is governed by the more ancient pathways of the complement system, our findings have multiple other implications, including:
a) The use of whole blood rather than blood plasma to test an individual's lipid profile
b) The use of assays for complement components MBL, C4 (C4A and C4B), C2, factors B and D, vitronectin, clusterin, complement split product C3adesArg, serum carboxypeptidase N (sCP-N), and erythrocyte-bound complement receptor 1 (CR1) to diagnose and further classify atherosclerosis and underlying or related disease(s)
c) The use of fresh-frozen plasma or either purified or recombinant human complement components, or human complement split product C3adesArg to substitute for the established deficiencies
d) The use of gene therapy for similar substitution purposes
e) The use of amphiphilic (partially lipophilic, partially hydrophilic) complement activators to bypass deficiencies in early complement components (MBL, C4A and C4B). It has recently been described that the half-life of MBL *in vivo* is acceptably long [97]
f) The diagnosis and adequate treatment of concomitant (infectious, autoimmune, or neoplastic) diseases that may (partially) occupy the lipid elimination pathway in order to prevent atherogenic processes
g) The prevention or treatment of exacerbations of paroxysmal nocturnal hemoglobinuria (PNH; a lipid-induced hemolytic disease) with a low-fat diet
h) The use of the body's lipid transportation system to efficiently manipulate the immune system, ass well as concomitant infectious, autoimmune, or hematologic diseases
i) The use of the lipid transport system to achieve optimum systemic immunosuppression by lipophilic immunosuppressants
j) The use of extracorporeal circulation and washing of (red) blood cells in subjects with serious hyperlipidemia.

### Conclusions

On the basis of the above-mentioned, we have arrived at the following conclusions:
1. The primary function of complement is the regulation of lipid metabolism rather than to complement or sustain the activities of the immune system; most other activities are secondary to this main function.
2. Disturbances in chylomicron- and/or chylomicron-remnant-mediated complement activation lead to impaired lipid metabolism.
3. Disturbances in the complement cascade, albeit subtle, may lead to impaired lipid metabolism and, on the long term, to atherosclerosis.
4. Correction of disturbed complement function, in case of impaired complement-dependent lipid metabolism, leads to an amelioration of lipid metabolism.
5. Correction of disturbed complement function, in case of impaired complement-dependent lipid metabolism, leads to an amelioration of disorders associated with impaired chylomicron remnant clearance.
6. Correction of disturbed complement function, in case of impaired complement-dependent lipid metabolism, leads to an amelioration of atherosclerosis and underlying or otherwise related diseases (FCHL, insulin resistance in association with type-2 diabetes and/or obesity, or coronary heart disease/premature atherosclerosis).
7. Disturbed chylomicron clearance in case of apo B48 hypoglycosylation can be overcome by the local application of glycosylation enzymes, their encoding gene(s) and/or gene fragments.
8. The clinical use of purified or recombinant human mannose-binding lectin (huMBL), C4A, C4B, C3adesArg, serum carboxypeptidase N, clusterin and/or vitronectin, or the encoding genes or fragments of these genes in diseases with disturbed lipid metabolism or underlying / related defects (*e.g.* infectious, or systemic autoimmune, or neoplastic disease).
9. The clinical use of amphiphilic complement activators in diseases with disturbed lipid metabolism or underlying / related defects.
10. The clinical use of natural or (semi)synthetic, amphiphilic complement activators in diseases with disturbed lipid metabolism or underlying / related defects.
11. The clinical use of oils containing critical concentrations of (a) natural or (semi)synthetic, amphiphilic complement activator(s) as food additive(s) for patients with disturbed lipid metabolism or underlying / related defects.
12. The clinical use of phytosterols in diseases with disturbed lipid metabolism or underlying / related defects.
13. The use of phytosterol-containing, purified or chemically modified mineral and/or natural oils as food additives for patients with disturbed lipid metabolism.
14. The use of oils containing a critical concentration of phytosterol(s) as food additives in patients with disturbed lipid metabolism or underlying / related disease.
15. The clinical use of lipophilic tertiary or quaternary ammonium compound(s) in diseases with disturbed lipid metabolism or underlying / related disease.
16. The use of lipophilic tertiary or quaternary ammonium-compound-containing purified and/or chemicically modified mineral or natural oil(s) as (a) food additive(s) for patients with disturbed lipid metabolism or underlying / related disease.
17. The use of purified and/or modified, mineral or natural oils containing (a) critical concentration(s) of (a) lipophilic tertiary or quaternary ammonium compound(s) as (a) food additive(s) for patients with disturbed lipid metabolism.
18. The application of purified and/or chemically modified, mineral or natural oil(s) containing (a) critical concentration(s) of lipophilic, tertiary or quaternary ammonium compound(s) as vehicula for oral vaccines.
19. The use of purified and/or chemically modified mineral or natural oils enriched with a critical concentration of lipidated muramyl-dipeptide as vehicula for oral vaccines.
20. The use of purified and/or chemically modified mineral or natural oils containing amphiphilic complement activators as lymph-targeting, oro-mucosal adjuvants to induce enhanced mucosal antibody (IgA) responses, T-cell reactivity, and/or systemic (IgM and/or IgG) antibody responses.
21. The use of purified and/or modified mineral and/or natural containing lipidated low-Mr dextran sulphate as lymph-targeting, oro-mucosal adjuvant to induce enhanced mucosal (IgA) and/or systemic T-cell or B-cell (IgM and/or IgG antibody) responses.
22. The estimation of complement activation/consumption by plant-derived, synthetic, or semisynthetic substances as a parameter of their anti-atherogenic potential.
23. The estimation of the next complement components: MBL, C4A, C4B, C2, factor B, C3adesArg, serum carboxypeptidase N, vitronectin, clusterin, and erythrocyte-bound complement receptor 1 (CR1) in blood, blood serum and/or blood plasma of a patient in order to establish the underlying or related defect of his/her atherosclerosis.

### References:

1. Ross R. The pathogenesis of atherosclerosis: a perspective for the 1990's. Nature 1993; 362: 801-809.
2. Grundy SM, Wilhelmsen L, Rose G, Campbell RWF, Assman G. Coronary heart disease in high-risk populations: Lessons from Finland. Eur Heart J 1990; 11: 462-471.
3. Willeit J, Kiechl S, Oberhollenzer F, Rungger G, Egger G, Bonora E, Mitterer M, Muggeo M. Distinct risk profiles of early and advanced atheroclerosis. Prospective results from the Brunneck Study. Arterioscl Thromb Vasc Biol 2000; 20: 529-537.
4. Rosamond WD, Chambless LE, Folsom AR, Cooper LS, Conwill DE, Clegg LC, Wang CH, Heiss G. Trends in the incidence of myocardial infarction and in mortality due to coronary heart disease, 1987 to 1994. N Engl J Med 1998; 339: 861-7.
5. Bucher HC, Griffith LE, Guyatt GH. Systematic review on the risk and benefit of different cholesterol-lowering interventions. Arterioscl Thromb Vasc Biol 1999; 19: 187-195.
6. The Heart Outcmes prevention Evaluation Study Investigators. Effects of an angiotensin-converting-enzyme inhibitor, ramipril, on cardiovascular events in high-risk patients. N Engl J Med 2000; 342:145-53.
7. The Diabetes Control and Complication Trial Research Group. The effect of intensive treatment of diabetes on the development and progression of long-term complications in insulin-dependent diabetes mellitus. N Engl J Med 1993; 329: 977-86.
8. UK Prospective Diabetes Study (UKPDS) Group. Intensive blood-glucose control with sulphonylureas or insulin compared with conventional treatement and risk of complications in patients with type 2 diabetes (UKPDS 33). Lancet 1998; 352:837-53.
9. Haffner SM, Lehto S, Ronnemaa T, Pyorala K, Laakso M. Mortality from coronary heart disease in subjects with type 2 diabetes and in nondiabetic subjects with and without prior myocardial infarction. N Engl J Med 1998; 339: 229-34.
10. Ross R. Atherosclerosis: an inflammatory disease. N Engl J Med 1999; 340: 115-126.
11. Kuller LH, Tracy RP, Shaten J, Meilahn EN. Relation of C-reactive protein and coronary heart disease in the MRFIT nested case-control study. Am J Epidemiol 1996; 144: 537-547.
12. Ridker PM, Cushman M, Stampfer MJ, Tracy RP, Hennekens CH. Inflammation, aspirin, and the risk of cardiovascular disease in apparently healthy men. N Engl J Med 1997; 336: 973-979.
13. Koenig W, Sund M, Frohlich M, Fischer H-G, Lowell H, Doring A, Hutchinson WL, Pepys MB. C-reactive protein, sensitive marker of inflammation, predicts future risk of coronary heart disease in initially healthy middle-aged men. Circulation 1999; 99: 237-242.
14. Strandberg TE, Tilvis RS. C-reactive protein, cardiovascular risk factors, and mortality in a prospective study in the elderly. Arterioscl Thromb Vasc Biol 2000; 20: 1057-1060.
15. Law SKA & KBM Reid: Complement in focus, 2nd edition, 1995. Oxford University Press, Oxford, G.B.
16. Mathews KP, PM Pan, NJ Gardner & TE Hugli: Familial carboxypeptidase N deficiency. Ann Intern Med 93 (1980) 443-5.
17. Plow EF, K Allampallam & A Redlitz: The plasma carboxypeptidases and the regulation of the plasminogen system. Trends Cardiovasc Med 7 (1997) 71-5.
18. Siegl J, TL Liu & N Gleicher: The red cell immune system. Lancet ii (1981) 556-9.
19. Sunyer JO, IK Zarkadis & JD Lambris: Complement diversity: a mechanism for generating immune diversity? Immunol Tod 19 (1998) 519-23.
20. Kocourek J & V Horejsi: Defining a lectin. Nature 290 (1981) 188.
21. Super M, S. Thiel, J Lu, RJ Levinsky & MW Turner: Association of low levels of mannan-binding protein with a common defect of opsonisation. Lancet 2 (1989) 1236-9.
22. Hansen S & U Holmskov: Structural aspects of collectins and receptors for collectins. Immunobiology 199 (1998) 165-89.
23. Galanos C, Eth Rietschel, O Lüderitz & O Westphal: Interaction of lipopoly-saccharides and lipid A with complement. Eur. J. Immunol. 19 (1971) 143-52.
24. Loos M, D Bitter-Suermann & M. Dierich: Interaction of the first (C1), and the fourth (C4) component of complement with different preparations of bacterial lipopolysaccharides and with lipid A: J. Immunol.: 112 (1974) 935-40.
25. Cooper NR & DC Morrison: Binding and activation of the first component of human complement by the lipid A region of lipopolysaccharides. J. Immunol.. 120 (1978) 1862-8.
26. Taylor PW: Antibacterial and bacteriolytic activity of serum against gram-negative bacteria. Microbiol. Rev. 47 (1984) 46-83.
27. Tenner AJ, RJ Ziccaridi & NR Cooper: Antibody-independent C1 activation by E. coli. J. Immunol. 133 (1984) 886-91.
28. Grossman N, KA Joiner, MM Frank & L Leive: C3b binding, but not its breakdown, is affected by the structure of the O-antigen polysaccharidein lipopolysaccharide from salmonellae. J. Immunol. 136 (1986) 2208-15.
29. Loos M & F Clas: Antibody-independent killing of gram-negative bacteria via the classical pathway of complement. Immunol. Lett 14 (1986/7) 203-8.
30. Kroes BH, CJ Beukelman, AJJ van den Berg, GJ Wolbink, H van Dijk & RP Labadie: Inhibition of human complement by β-glycyrrhetinic acid. Immunology 90 (1997) 115-20.
31. Yamada HY, M Yoshino, T Matsumoto, T Nagai, H Kiyohara, J-C Cyong, A Nakagawa, H Tanaka & S Omura: Effects of phytosterols on anticomplementary activity. Chem. Pharm. Bull. 35 (1987) 4851-5.
32. Bitter-Suermann D, R. Burger & U Hadding: Activation of the alternative pathway of complement: efficient fluid-phase amplification by blockade of the regulatory complement protein β1H through sulfated polyanions. Eur. J. Immunol. 11 (1981) 291-5.
33. Williams DL, ER Sherwood, RB McNamee, EL Jones & NR Di Luzio: Therapeutic efficacy of glucan in a murine model of hepatic metastatic disease. Hepatology 5 (1985) 198-206.
34. Di Luzio NR: Update on the immunomodulating activities of glucans: Springer Semin. Immunopathol. 8 (1985) 387-400.
35. Adam A & E Lederer: Muramyl peptides: Immunomodulators, sleep factors, and vitamins. Med. Res. Reviews 4 (1984) 111-52.
36. Kobayashi S, T Fukuda, H Yukimasa M Fujino, I Azuma & Y Yamamura: Synthesis, and the adjuvant and tumor-suppressive activities of quinonyl muramyl dipeptides. Bull. Chem. Soc. Jpn. 57 (1984) 3182-96.
37. Klerx JPAM, AJM van Oosterhout & H van Dijk: Anticomplementary amines are immunological adjuvants in mice. Immunol. Lett. 10 (1985) 281-5.
38. Castro Cabezas M & DW Erkelens: Triglycerides and atherosclerosis: to feast or fast. Neth J Med 56 (2000) 110-8.
39. Sasak WV, JS Lown & KA Colburn: Human small intestine apolipoprotein B48 oligosaccharide chains. Biochem J 274 (1991) 159-65.
40. Mahley RW, TL Innerarity, SC Rall & KH Weisgraber: Plasma lipoproteins: apolipo-protein structure and function. J Lipid Res 23 (1984) 1277-94.
41. Mahley RW, DY Hui, TL Innerarity & U Beisiegel: Chylomicron-remnant metabolism: role of hepatic lipoprotein receptors in mediating uptake. Arteriosclerosis 19S (1989) I14-8.
42. Hussain MM, FR Maxfield, J Más-Olivas,I Tabas, Z-S Ji, TL Innerarity & RW Mahley: Clearance of chylomicron remnants by the low-density receptor-related protein/α2-macroglobulin receptor. J Biol Chem 266 (1991) 13936-40.
43. Luoma J, T Hiltunen, T Särkioja, SK Moestrup, J Gliemann, T Kodama, T Nikkari & S Ylä-Herttuala: Expression of α2-macroglobulin receptor/low-density lipoprotein receptor-related protein and scavenger receptor in human atherosclerotic lesions. J Clin Invest 93 (1994) 2014-21.
44. Van Dijk MCM, GJ Ziere, W Boers, C Linhorsts, MK Bijsterbosch & TJC van Berkel: Recognition of chylomicron remnants and β-migrating very-low-density lipoproteins by the remnant receptor of parechymal liver cells is distinct from the liver α2-macroglobulin-recognition site. Biochem J 279 (1991) 863-70.
45. Windler E, J Greeve, B Levkau, V Kolb-Bachofen,W Daerr & H Greten: The human asialoreceptor is a possible binding site for low-density lipoproteins and chylomicron remnants. Biochem J 276 (1991) 79-87.
46. Mann CJ, J Khallou, O Chevreuil, A Troussard, LM Guermani, K Launay, B Delplanque, FT Yen & BE Bihain: Mechanism of activation and functional significance of the lipolysis-stimulated receptor. Evidence for a role as chylomicron remnant receptor. Biochem J 34 (1995) 10421-31.
47. Jäckle S, F Rinninger, J Greeve, H Greten, & E Windler: Regulation of the hepatic removal of chylomicron remnants and β-very-low density lipoproteins in the rat. J Lipid Res 33 (1992) 419-29.
48. Niemeier A, M Gåfels, J Heeren, B. Angelin & U Beisiegel: VLDL receptor mediates the uptake of human chylomicron remnants in vitro. J Lipid Res 37 (1996) 1733-42.
49. Esterbauer H, Krempler, F, Oberkofler H & Patch W: The complement system: a pathway linking host defence and adipocyte biology. Eur J Clin Invest 29 (1999) 653-656.
50. Maslowska M, Vu H, Sniderman AD, Rhode BM, Blank D & Cianflone K: Plasma acylation-stimulating protein, adipsin, and lipids in non-obese and obese populations. Eur J Clin Invest 29 (1999) 679-86.
51. Zilversmit DB: Atherogenesis: a postprandial phenomenon. Circulation 60 (1979) 473-85.
52. Whitman SC, CG Sawyez, DB Miller, BM Wolfe & MW Huff: oxidized type-IV hypertriglyceridemic VLDL remnants cause greater macrophage cholesteryl ester accumulation than oxidized LDL. J Lipid Res 39 (1998) 1008-20.
53. Castro Cabezas M & DW Erkelens: The direct way from gut to vessel wall: Atheroinitiation. Eur J Clin Invest 28 (1998) 504-5.
54. Castro Cabezas M, TWA de Bruin & DW Erkelens: Familial combined hyperlipidemia: 1973-1991. Neth J Med 40 (1992) 83-95.
55. Castro Cabezas M, TWA de Bruin, & HW de Valk *et al.*: Impaired fatty acid metabolism in familial combined hyperlipidemia. A mechanism associating hepatic apolipoprotein B overproduction and insulin resistance. J Clin Invest 1993; 92: 160-8.
56. Castro Cabezas M, TWA de Bruin, H Jansen, LAW Kock, W Kortlandt & DW Erkelens: Impaired chylomicron remnant clearance in familial combined hyperlipidemia. Arterioscler Thromb 13 (1993) 804-14.
57. Van Wanrooy F, R Stolk, M Castro Cabezas & Erkelens DW: Diabetic dyslipidemia: metabolic and epidemiological aspects. In: Current Perspectives in Diabetes. D.J. Betteridge, editor; Martin Dunitz Ltd, London. 1999; pages: 125-39.
58. Castro Cabezas M, TWA de Bruin, HE Westerveld, E Meijer, LAW Kock & DW Erkelens: Delayed chylomicron remnant clearance in subjects with heterozygous familial hypercholesterolemia. J Intern Med 244 (1998) 299-307.
59. Castro Cabezas M, TWA de Bruin, LAW Kock *et al*.: Postprandial apolipoprotein B100 and apo B48 in Familial Combined Hyperlipidemia before and after reduction of fasting plasma triglycerides. Eur J Clin Invest 24 (1994) 669-78.
60. Mahley RW & Z-S Ji: Remnant lipoprotein metabolism: key pathways involving cell-surface heparan sulfate proteoglycans and apolipoprotein E. J. Lipid Res. 40 (1999) 1-16.
61. Innerarity TL, Mahley RW, Weisgraber KH, Bersot TP et al. Familial defective apolipoprotein B-100: a mutation of apolipoprotein B that causes hypercholesterolemia. J Lipid Res 1990; 31: 1337-1349.
62. Westerveld HE, E Meyer, TWA de Bruin, & DW Erkelens: Oestrogens and postprandial lipid metabolism. Biochem Soc Transact 25 (1997) 45-9.
63. Baggio G, Manzato E, Gabelli C, *et al.* Apolipoprotein C-II deficiency syndrome. Clinical features, lipoprotein characterization, lipase activity, and correction of hypertriglyceridemia after apolipoprtein C-II administration in two affected patients. J Clin Invest 1986; 77: 520-527.
64. Goldberg IJ. Lipoprotein lipase and lipolysis: central roles in lipoprotein metabolism and atherogenesis. J Lipid Res 1996; 37: 693-707.
65. Hegele RA, Little A, Vezian C, Maguire GF, Tu L, Wolever TS, Jenkins DJA, Connelly PW. Hepatic lipase deficiency. Clinical, biochemical and molecular genetic characteristics. Arterioscl Thromb 1993; 13: 720-728.
66. Halkes CJM, M Castro Cabezas, PPTh de Jaegere, HWM Plokker & DW Erkelens: Expanded dose simvastatin improves postprandial lipoprotein metabolism in normolipidemic male patients with premature coronary sclerosis. First Conference on Arterioscl Thromb Vasc Biol; Denver May 20-22, 2000.
67. Simons LA, T Dwyer, J. Simons, L Bernstein, P Mock NS Poonia, S Balausbramaniam, D Baron, J Branson, J Morgan & P Roy: Chylomicrons and chylomicron remnants in coronary artery disease: a case-control study. Atherosclerosis 65 (1987) 181-9.
68. Patsch JR, G Miesenbock, T Hopferwieser, V Muhlberger, E Knapp, JK Dunn,,AM Gotto Jr & W Patsch: Relation to triglyceride metabolism and coronary artery disease. Studies in the postprandial state. Arterioscl Thromb 12 (1992) 1336-45.
69. Weintraub MS, I Grosskopf, T Rassin, H Miller, G Charach, HH Rotmensch, M Liron, A Rubinstein,& A Iaina A: Clearance of chylomicron remnants in normolipidaemic patients with coronary artery disease; case control study over three years. Br Med J 312 (1996) 935-9.
70. Ryu JE, G Howard, TE Craven, MG Bond & AP Hagaman, Crouse JR III: Postprandial triglyceridemia and carotid atherosclerosis in middle-aged subjects. Stroke 23 (1992) 823-8.
71. Karpe F, P Tornvall, T Olivecrona T, G Steiner, LA Carlson, A Hamsten: Postprandial lipoproteins and progression of coronary atherosclerosis. Atherosclerosis 106 (1994) 83-97.
72. Meyer E, HW Westerveld, DW Erkelens et al: Abnormal postprandial apolipoprotein B48 and triglyceride responses in normolipidemic women with greater than 70 percent stenotic coronary artery disease. A case control study. Atherosclerosis 124 (1996) 221-35.
73. Groot PHE, WAHJ van Stiphout, XH Krauss, H Jansen, A van Tol, E van Ramshorst, S Chin-On, SR Cresswell & L Havekes: Postprandial lipoprotein metabolism in normolipidemic men with and without coronary artery disease. Arterioscler Thromb 11 (1991) 653-62.
74. Ginsberg HN, J Jones J, WS Blaner, *et al.* Association of postprandial triglyceride and retinyl palmitate response with newly diagnosed exercise-induced myocardial ischemia in middle-aged men and women. Arterioscl Thromb Vasc Biol 15 (1995) 1829-38.
75. Cianflone KM, AD Sniderman, MJ Walsh, HT Vu, J Gagnon & MA Rodriguez: Purification and characterization of acylation-stimulating protein. J Biol Chem 264 (1989) 426-30.
76. Baldo A, AD Sniderman & S St-Luce, *et al.*: The adipsin-acylation-stimulating protein system and regulation of intracellular triglyceride synthesis. J. Clin. Invest. 92 (1997) 1543-7.
77. Newkirk MM, P Persefoni, C Neville & PR Fortin: Systemic lupus erythematosus, a disease associated with low levels of clusterin/apo J, an antiiflammatory protein. J Rheumatol 26 (1999) 597-603.
78. Nelson DS: Immune adherence. In'Ciba Foundation Symposium on Complement' (GEW Wolstenholme and J Knight, eds). J&A Churchill Ltd, London, pp. 222-37.
79. Varela P, N Slobodianik, A Pallaro, A Marcos, S Barbeito, P Taberner, P Marino, A Franchello & O Ramos: Some nutritional parameters in adolescent females suffering from obesity or anorexia nervosa. World Rev Nutr Diet 82 (1997) 168-174.
80. Ylitalo K, KV Porkka, S Meri, I Nuotio, L Suurinkeroinen, J Vakkilainen, P Pajukanta, JS Viikari, L Peltonen, C Ehnholm & MR Taskinen: Serum complement and familial combined hyperlipidemia. Atherosclerosis 129 (1997) 271-7.
81. Muscari A, G Massarelli, L Bastagli, G Poggiopollini, V Tomasetti, U Volta, GM Puddu & P Puddu: Relationship between serum C3 levels and traditional risk factors for myocardial infarction. Acta Cardiol 53 (1998) 345-54.
82. Swaminathan N & F Aladjem: The monosaccharide composition and sequence of the carbohydrate moiety of human serum low density lipoproteins. Biochemistry 15 (1976) 1516-22.
83. Daha MR: Autoantibodies against complement components. In 'Complement in health and disease' (K Whaley, editor). MTP Press Ltd (Kluwer group), Lancaster (1986) pp. 185-99.
84. Harmsen T, S Kuipers, EM Mascini & H van Dijk: Cross-sectional study on soluble terminal-complement pathway inhibitor levels in the Dutch population. Immunopharmacol. 49 (2000) 21.
85. Carroll MC, RD Campbell, DR Bentley & RR Porter: a molecular map of the human histocompatibility complex class III region linking complement genes C4, C2, and factor B. Nature 307 (1984) 237-41.
86. Kuipers S, T. Harmsen, E. Mascini, K Takahashi & H. van Dijk: Mannose-binding lectin in The Netherlands: prevalence of normal and aberrant levels in a healthy population. Immunopharmacol. 49 (2000) 87.
87. Orlean P: Congenital disorders of glycosylation caused by defects in mannose addition during N-linked oligosaccharide assembly. J Clin Invest 105 (2000) 131-32.
88. Rowe PC, RH McLean, RA Wood, RJ Leggiadro & JA Winkelstein: Association of homozygous C4B deficiency with bacterial meningitis. J. Infect. Dis. 160 (1989) 448-51.
89. Bishof NA, TR Welch & LS Beischel: C4B deficiency: a risk factor for bacteremia with encapsulated organisms. J. Infect. Dis. 162 (1990) 248-50.
90. Fasano MB, P. Densen, RH mclean & JA Winkelstein: Prevalence of homozygous C4B deficiency in patients with deficiencies of terminal complement components and meningococcemia. J. Infect. Dis. 162 (1990) 1120-21.
91. Hartung K, MP Baur, R Coldeway, M Fricke, JR Kalden, HJ Lakomek, HH Peter, D Schendel, PM Schneider, SA Seuchter, W Stangel & HRG Deicher: Major histocompatibility complex haplotypes and complement C4 alleles in systemic lupus erythematosus. J. Clin. Invest. 90 (1992) 1346-51.
92. Yu CY, CA Blanchong, B Zhou, L Rupert, Z Yang, KN Jones & RM Rennebohm: The 1-2-3 loci theory of human complement component C4: heterozygosity in gene size and gene number as a mechanism to maintain C4 genetic diversity in the population. Immunopharmacol. 49 (2000) 32.
93. Birmingham DJ, G Liang & X-P Shen: CR1 polymorphism associated with SLE. Immunopharmacol. 49 (2000) 29.
94. Borba EF, E Bonfa, CG Vinagre, JA Ramires & RC Maranhao: Chylomicron metabolism is markedly altered in systemic lupus erythematosus. Arthritis Rheum. 43 (2000) 1033-40.
95. Van Damme EJM, WJ Peumans, A Pusztai & S Batdocz: Handbook of plant lectins: properties and biomedical applications. John Wiley & Sons, Chichester, GB (1997) 8.
96. Ng TB, WW Li & HW Yeung: Effects of lectins with various carbohydrate binding specificities on lipid metabolism in isolated rat and hamster adipocytes. Int. J. Biochem. 21 (1989) 149-55.
97. Valdimarsson H, M Stefansson, T Vikingsdottir, GJ Arason, C Koch, S Thiel & JC Jensenius: Reconstitution of opsonizing activity by infusion of mannan-binding lectin (MBL) to MBL-deficient humans. Scand. J. Immunol. 48 (1998) 116-23.

## Claims

1. Pharmaceutical composition for the treatment or prophylaxis of atherosclerosis or an underlying and/or related disease, which composition comprises one or more of human or recombinant human complement proteins chosen from the group of: MBL, C4A, C4B, C2, alternate-complement-pathway components B and D, C3adesArg, serum carboxypeptidase N (sCP-N), vitronectin, clusterin (Apo J), erythrocyte-bound or free complement receptor 1 (CR1), encoding genes thereof and fragments thereof.

2. Composition according to claim 1 comprising apolipoprotein B (48 or 100), a (semi)synthetic mimetic of apo B, or (an)other amphiphilic (= partially hydrophobic and partially hydrophilic) ligand(s) of mannose-binding lectin (e.g. mannosylated, N-acetylglucosaminylated, and/or fucosylated phytosterols).

3. Composition according to claim 2, further comprising an activator chosen from the groups of natural lipo-oligogosaccharides, lipopolysaccharides, lipidated oligo- or polysaccharides, β-glycyrrhetinic acid, phytosterols (β-sitosterol, campesterol, and/or stigmasterol), stannols, lipidated dextran sulphate(s), (lipo)glucan(s), lipidated tertiary or quaternary ammonium compounds, combinations thereof, and single and/or combined related substances.

4. Composition according to claim 1 comprising an IgA or IgD antibody, which is heavily mannosylated of either polyclonal or humanised monoclonal or combinatorial origin, directed towards one of the apolipoproteins of chylomicrons or very low-density lipoproteins (VLDL).

5. Composition according to claim 1 comprising an amphiphilic partially hydrophobic or hydrophilic complement activator of natural or (semi)synthetic origin or a mimetics of such a complement activator.

6. Composition according to claims 2-5, in which the complement activator(s) primarily act(s) on the lectin complement activation pathway.

7. Composition according to claims 2 -5, wherein the complement activator(s) help(s) to bypass the lectin complement activation pathway.

8. Composition according to claims 4 and 5, in which the complement activator(s) act(s) primarily on the alternative complement activation pathway.

9. Composition according to claim 1 comprising one or more soluble or lipidated complement inhibitors of natural or (semi)synthetic origin (e.g. sialylated glycolipids).

10. Composition according to any of the preceding claims, further comprising a pharmaceutically acceptable carrier.

11. _ Composition according to claim 10, wherein the pharmaceutically acceptable carrier is chosen from the group of mineral, natural or processed (purified and/or modified) mineral or natural oils.

12. Use of a composition according to any of the preceding claims for the treatment and/or prevention of atherosclerosis or underlying / related disease.

13. Use according to any of the preceding claims, in which the natural or (semi)sythetic complement activator or complement inhibitor has been produced by combinatorial strategies.

14. Use according to any of the preceding claims, wherein the natural or (semi)synthetic complement activator or inhibitor is generated *in vivo (e.g.* by gene therapy).

15. Use of at least one complement activator and/or complement inhibitor for the manufacture of a medicament for the treatment and/or prevention of atherosclerosis or underlying / related disease.

16. Use of an *in-vitro* assay for the estimation of complement activation/consumption by natural or (semi)synthetic substances as a parameter of their anti-atherogenic potential.

17. Method for diagnosing atherosclerosis or underlying or related disease by carrying out an assay for at least one activatory or regulatory complement component.

18. Method according to claim 16, wherein the assay is carried out for the detection of at least one natural complement activator chosen from the group of: apo B (apo B48, apo B100, or both), MBL, C4, C4A, C4B, C2, factor B, factor D, vitronectin, clusterin, erythrocyte-bound CR1, C3adesArg, carboxypeptidase-N, and chylomicron-bound sialic acid.

19. Kit for diagnosing atherosclerosis or underlying / related disease by a method according to claim 17 or 18, comprising means for carrying out an assay for at least one natural activatory or inhibitorys complement component.

20. Use of a lipidated vaccine for oral immunisation.

21. Use according to claim 20, wherein the lipidated vaccine comprises one or more immunological adjuvants chosen from the group of lipidated tertiary or quaternary ammonium compounds, lipidated muramyldipeptide, sialylated glycolipids, lipidated dextran sulphates, plant saponins or fractions of plant saponins or a combination of these adjuvants.

22. Use of whole blood or erythrocytes instead of blood plasma as a substrate for estimating a patient's lipid profile.
A method for treating paroxysmal nocturnal hemoglobinuria by keeping a diet low in fats.
